(19) Europäisches Patentamt / European Patent Office / Office européen des brevets

(11) **EP 4 455 710 B1**

(12) **EUROPEAN PATENT SPECIFICATION**

(45) Date of publication and mention
of the grant of the patent:
**06.05.2026 Bulletin 2026/19**

(21) Application number: **24172772.6**

(22) Date of filing: **26.04.2024**

(51) International Patent Classification (IPC):
*G01R 33/565* (2006.01)    *G01R 33/24* (2006.01)

(52) Cooperative Patent Classification (CPC):
**G01R 33/5659; G01R 33/246**

(54) **SHIMMING METHOD AND SHIMMING APPARATUS**

SHIMVERFAHREN UND SHIMVORRICHTUNG

PROCÉDÉ DE CALAGE ET APPAREIL DE CALAGE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **28.04.2023 US 202318309210**

(43) Date of publication of application:
**30.10.2024 Bulletin 2024/44**

(73) Proprietor: **Canon Medical Systems Corporation
Tochigi 324-0036 (JP)**

(72) Inventors:
• **WHEATON, Andrew James
Otawarashi, 324-0036 (JP)**
• **DANNELS, Wayne R.
Otawara-shi, 324-0036 (JP)**

(74) Representative: **Marks & Clerk LLP
15 Fetter Lane
London EC4A 1BW (GB)**

(56) References cited:
**EP-A1- 4 170 372    US-A1- 2016 282 438
US-B2- 9 086 446**

**Description**

FIELD

Embodiments described herein relate generally to a shimming method and shimming apparatus.

This disclosure relates to patient-specific shimming of radio frequency (RF) magnetic fields (i.e., "B1 fields") in magnetic resonance imaging (MRI) systems. The B1 field shimming is performed *in vivo* based on projection data acquired along one or more projections over a patient that are determined in accordance with the anatomy of the patient to be imaged.

BACKGROUND

The background description provided herein is for the purpose of generally presenting the context of the disclosure.

As part of the procedure for producing MRI images within the body of a patient, a static magnetic field (B0) is used by an MRI scanner to align the nuclear spins of atoms. During the scan, RF pulses generated by an RF transmitter cause perturbations to the local magnetic field, and RF signals emitted by the nuclear spins are detected by an RF receiver.

In order to achieve diagnostic images with high spatial resolution and high contrast resolution, the strength of B0 fields is increasingly higher (from 1.5T to 3T and above) in clinical practice. Under higher B0 fields, however, RF behavior in the patient becomes more complex. For example, the dielectric properties of the human body can cause local perturbations to the B1 fields, resulting in non-uniform excitation. This can introduce errors in the contrast of resultant diagnostic images, potentially leading to misdiagnosis.

To tackle this problem, a common approach is to map the RF transmit magnetic field and perform shimming correction in accordance with the map, so as to obtain a more uniform B1 field distribution. In a spatially resolved B1 map, each pixel represents a measurement of the transmit magnetic field B1 at that location. Besides B1 shimming, the map can also be used for RF transmit calibration (for accurate RF pulse flip angles), parallel transmit (pTx) RF pulse control (pTx is generally necessary for 7T MRIs, for example), and correction of quantitative relaxometry maps (commonly associated with longitudinal relaxation time (T1) mapping). A B1 map can be acquired as a pre-scan procedure, and then the B1 map can be used for calibration, design, or correction of data in subsequent sequence acquisitions during the protocol.

US9086446 discloses a method of B1 field mapping relating to Magnetic resonance imaging (MRI).

For the sake of saving scan time, it is beneficial for pre-scans to be as short as reasonably possible. Although there exist a number of B1 mapping approaches, a common downside is that the measurement time is impractically prolonged. A B1 shimming method that can be performed to correct the B1 field variation at clinical field strengths (e.g., 1.5T, 3T, etc.) within scan time on the order of one second has yet to be established.

Therefore, it is desirable to address these and other deficiencies of current approaches.

SUMMARY OF INVENTION

The invention is defined by the claims. Certain particular embodiments are presented in the dependent claims.

It is noted that B1 projection data is 1D image data obtained using gradient signals along the respective orientation for the projection.

BRIEF DESCRIPTION OF THE DRAWINGS

Various embodiments of this disclosure that are proposed as examples will be described in detail with reference to the following figures, wherein like numerals reference like elements, and wherein:

FIG. 1 shows exemplary dielectric artifacts in MRI images;

FIG. 2 shows exemplary B1 field maps acquired in four different imaging anatomies, i.e., head, abdomen, breasts, and thighs;

FIG. 3 shows a non-limiting example of a block diagram of a B1 field shimming apparatus according to one embodiment of the present disclosure;

FIG. 4 shows a non-limiting example of a flow chart of a B1 field shimming method according to one embodiment of the present disclosure;

FIG. 5 shows a non-limiting example of a block diagram of projection determination circuitry that determines one or more projections along which projection data is acquired by the MRI system, according to one embodiment of the present disclosure;

FIG. 6 shows a non-limiting example of a flow chart of a process of determining one or more projections along which projection data is acquired by the MRI system, according to one embodiment of the present disclosure;

FIG. 7 shows a non-limiting example of different projections and projection data acquired along the projections respectively, according to one embodiment of the present disclosure;

FIG. 8 shows a non-limiting example of different projection combinations identified for different imaging anatomy, according to one embodiment of the present disclosure;

FIG. 9 shows a non-limiting example of a block diagram of shimming parameter resolving circuitry that resolves a set of shimming parameters based on projection data acquired, according to one embodiment of the present disclosure;

FIG. 10 shows a non-limiting example of a flow chart of a process that resolves a set of shimming parameters based on projection data acquired, according to one embodiment of the present disclosure;

FIG. 11 shows a non-limiting example of a block diagram of shimming parameter resolving circuitry that resolves a set of shimming parameters based on projection data acquired, according to one embodiment of the present disclosure;

FIG. 12 shows a non-limiting example of a flow chart of a process that resolves a set of shimming parameters based on projection data acquired, according to one embodiment of the present disclosure;

FIG. 13 shows a non-limiting example of acquiring projection profiles along two or more orientations, with respect to different sets of shimming parameters, according to one embodiment of the present disclosure;

FIG. 14 shows a non-limiting example of acquiring projection profiles along one or more orientations, with respect to different sets of shimming parameters, according to one embodiment of the present disclosure;

FIG. 15 shows a non-limiting example of a block diagram of shimming parameter resolving circuitry that resolves a set of shimming parameters based on projection data acquired, according to one embodiment of the present disclosure;

FIG. 16 shows a non-limiting example of a flow chart of a process that resolves a set of shimming parameters based on projection data acquired, according to one embodiment of the present disclosure; and

FIG. 17 is a schematic block diagram of a magnetic resonance imaging system, according to one embodiment of the present disclosure.

## DETAILED DESCRIPTION

The present disclosure relates to a method for performing patient-specific B1 field shimming in a magnetic resonance imaging system. The method includes obtaining patient information of a patient to be imaged by the magnetic resonance imaging system. The method also includes determining an orientation of a projection based on the obtained patient information. In addition, the method includes acquiring B1 projection data, using the magnetic resonance imaging system, along the determined orientation of the projection. Further, the method includes determining a set of B1 shimming parameters based on the acquired B1 projection data.

The disclosure additionally relates to an apparatus for performing patient-specific B1 field shimming in a magnetic resonance imaging system. The apparatus comprises processing circuitry that obtains patient information of a patient to be imaged by the magnetic resonance imaging system. The processing circuitry also determines an orientation of a projection based on the obtained patient information. In addition, the processing circuitry acquires B1 projection data, using the magnetic resonance imaging system, along the determined orientation of the projection. Further, the processing circuitry determines a set of B1 shimming parameters based on the acquired B1 projection data.

The following disclosure provides many different embodiments, or examples, for implementing different features of the provided subject matter. Specific examples of components and arrangements are described below to simplify the present disclosure. These are, of course, merely examples and are not intended to be limiting.

For example, the order of discussion of the different steps as described herein has been presented for clarity's sake. In general, these steps can be performed in any suitable order, according to the scope of the claims. Additionally, although each of the different features, techniques, configurations, etc. herein may be discussed in different places of this disclosure, it is intended that each of the concepts can be executed independently of each other or in combination with each other. Accordingly, the present disclosure can be embodied and viewed in many different ways.

Furthermore, as used herein, the words "a," "an" and the like generally carry a meaning of "one or more," unless stated otherwise.

When electromagnetic waves encounter an object in an MRI scanner, dielectric effects occur: the wavelength decreases, electrical current is generated, and wave reflection/ refraction develops at tissue interfaces. Under a high B0 field (3T and above), the homogeneity of the time-varying RF magnetic field (B1) will be highly dependent on the electrical properties of the object, e.g., the body of a patient. As standing wave currents flow in opposite directions from two sides of the patient, a pattern with destructive interference (dark areas) and constructive interference (bright areas) separated by on the order of quarter wavelengths is created. These abnormal bright and dark areas due to B1 field inhomogeneity are commonly labeled as "dielectric artifacts."

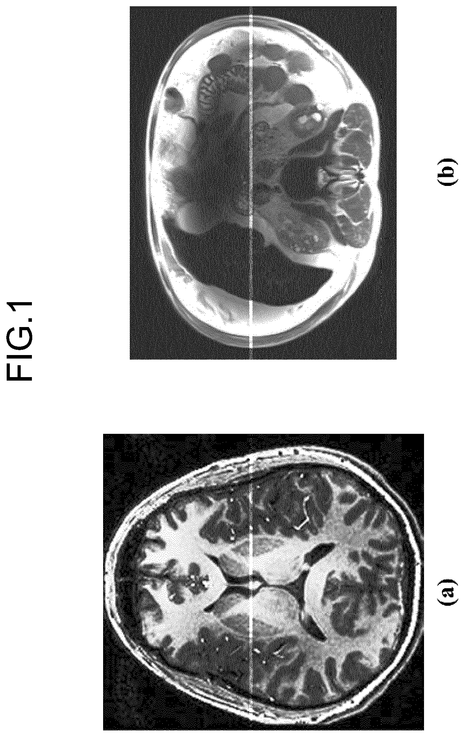

Portions (a) and (b) of FIG. 1 show exemplary dielectric artifacts in MRI images of human bodies. As can be seen in the portion (a) of FIG. 1, the center and anterior of the brain image is abnormality brightened. In the portion (b) of FIG. 1, the center of the abdomen image is abnormality darkened. These dielectric artifacts increase the difficulty of analyzing the MRI images and obtaining clinical information, and in severe cases, can lead to misdiagnosis.

To mitigate the dielectric artifacts, one approach is to acquire information representing the B1 field distribution for each patient such that B1 shimming can be conducted for each patient. This can improve homogeneity of the B1 field, and thus improve image quality and possibly the specific absorption rate (SAR) or B1 rms safety.

Many B1 shimming methods have been developed. Typically, these methods include acquiring a spatially resolved map using standard Cartesian phase encoding. Because B1 is slowly spatially varying, the encoding matrix can be small (corresponding to a low resolution). Commonly, the matrix sizes are $32\times32$, $48\times48$, or $64\times64$, for example. A single slice acquired at the isocenter is often used as a representation of the B1 distribution of the volume. At the cost of increased scan time, 3D B1 maps can also be acquired.

However, even with small matrix sizes (e.g., $32\times32$, $48\times48$, or $64\times64$), it takes several tens of seconds or even a few minutes to acquire data sufficient to build a B1 map. Even though certain fast methods (e.g., the Bloch-Siegert Shift method) can be used, it still requires 64 shots (32 phase encodes $\times$ 2 frequency offsets) to create a lowresolution B1 map. For example, at TR=200ms, it would take 12.8 seconds to acquired B1 data for each channel. When separate B1 maps are acquired for each of two channels, the total pre-scan time is about 26 seconds.

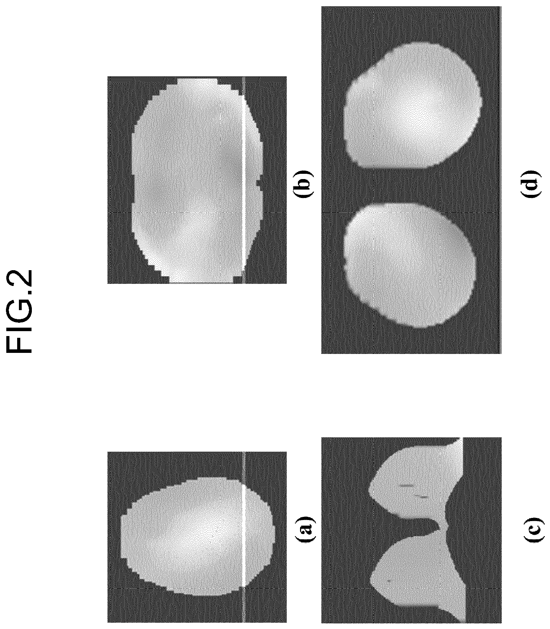

In the context of typical clinical B1 shimming, a fully Cartesian-encoded map is not necessary. Typically, there are only two control parameters: amplitude attenuation and phase offset $\{A, \varphi\}$ (i.e., settings for the TX2 channel relative to the TX1 channel, perhaps for two drive ports on a quadrature TX coil). Moreover, it is known that the B1 field is slowly spatially varying with a characteristic +/- pattern due to the dielectric effects. In addition, a rough estimate of the shape of the B1 field is known. Portions (a)-(d) of FIG. 2 show exemplary B1 field maps acquired for four different imaging anatomies, i.e., head, abdomen, breasts, and thighs. Each of the B1 maps illustrated in the portions (a)-(d) of FIG. 2 has a characteristic distribution, which corresponds to a +/- pattern caused by the dielectric effects. Individual patient maps fit similar characteristic patterns, differing in aspect ratio, spatial scaling, scaling of B1 nonuniformity, etc.

Therefore, when used for purposes of B1 shimming, a B1 map essentially contains no more useful information than a $2\times2$ image. By acquiring projection data instead of Cartesian encoding, sufficient information can be extracted from one or more 1D projections. In particular, the symmetry of the projection data can reveal adequate information for calculating the optimal $\{A, \varphi\}$ set. A map is not necessary in order to solve for $\{A, \varphi\}$; what is required is a few metrics of the B1 spatial distribution. In this way, by acquiring only a few (1, 2, 3, or 4, for example.) projections instead of 32 phase encodes, the scan time can be significantly reduced from tens of seconds to approximately one second.

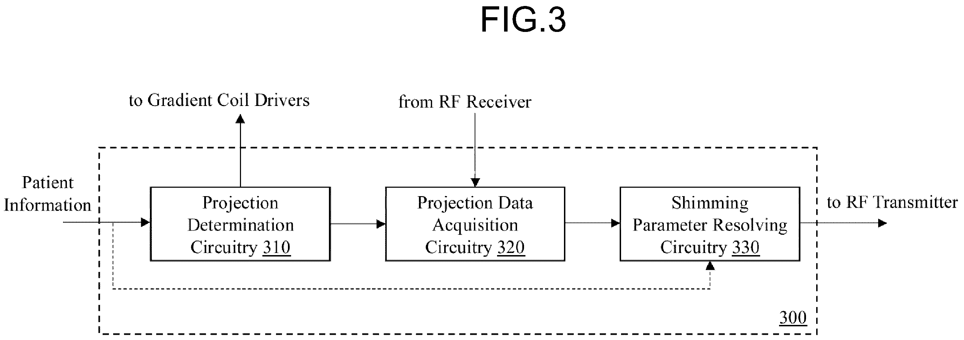

FIG. 3 shows a non-limiting example of a block diagram of a B1 field shimming apparatus according to one embodiment of the present disclosure. The B1 field shimming apparatus 300 includes projection determination circuitry 310, projection data acquisition circuitry 320, and shimming parameter resolving circuitry 330. The projection determination circuitry 310 obtains information (patient information) regarding the patient to be imaged, determines one or more projections along which projection data is to be acquired, determines gradient signals that are required to achieve the projections, and applies the gradient signals to gradient coil drivers of the MRI scanner. The projection data acquisition circuitry 320 acquires projection data from the RF receiver of the MRI scanner, and sends the projection data to the shimming parameter resolving circuitry 330. Based on the projection data (and the optional patient information), the shimming parameter resolving circuitry 330 resolves and outputs a set of shimming parameters to the RF transmitter of the MRI scanner.

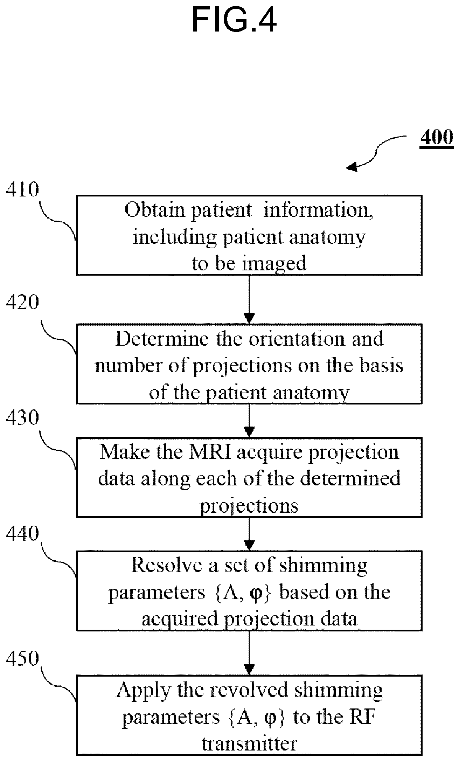

FIG. 4 shows a non-limiting example of a flow chart of a B1 field shimming method according to one embodiment of the present disclosure. The process 400 of the B1 field shimming method begins at step 410 by obtaining patient information. The patient information includes patient anatomy to be imaged. At step 420, the orientation and number of projections are determined based on the imaging anatomy. The projection orientations may initially be specified relative to patient anatomical axes, such as left-right, head-foot, and anteriorposterior. Among the patient information, there may be information about the orientation of the patient within the MRI-scanner, such as prone-versus-supine, and head-first positioning or feet-first positioning. The anatomical projection orientations plus the patient orientation are sufficient to define the projections in the scanner hardware coordinates, such as x, y and z gradient directions. This kind of geometric transformation is well known in MRI. At step 430, the MRI scanner is caused to acquire projection data along each of the determined projections. At step 440, a set of shimming parameters are resolved based on the acquired projection data. Optionally, the patient information is also used in resolving of the shimming parameters. At step 450, the resolved shimming parameters are applied to the RF transmitter to control individual transmit channels of the MRI scanner.

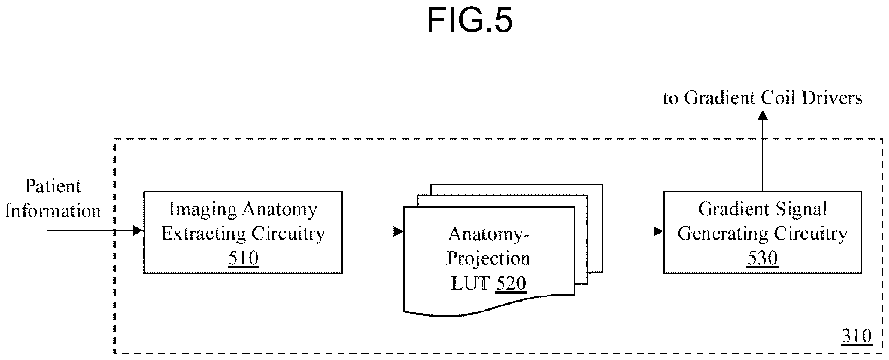

The components of FIG. 3 and the processes performed by those components will be described in more detail below with reference to FIGs. 5, 6, 9, 10, 11, 12, 15, and 16.

FIG. 5 shows a non-limiting example of a block diagram of projection determination circuitry that determines one or more projections along which projection data is acquired by the MRI scanner, according to one embodiment of the present disclosure. The projection determination circuitry 310 includes imaging anatomy extracting circuitry 510, anatomy-projection look-up table (LUT) 520, and gradient signal generating circuitry 530. The imaging anatomy extracting circuitry 510 receives information (patient information) regarding the patient and extracts the patient anatomy to be imaged. Many anatomy-projection pairs are prestored in the anatomy-projection look-up table 520. By comparing the

**EP 4 455 710 B1**

imaging anatomy against the entries stored in the anatomy-projection look-up table 520, one or more matched projections are searched out and sent to the gradient signal generating circuitry 530. Upon reception of the matched projections, the gradient signal generating circuitry 530 generates gradient signals that are required to achieve the projections and outputs them to the gradient coil drivers of the MRI scanner.

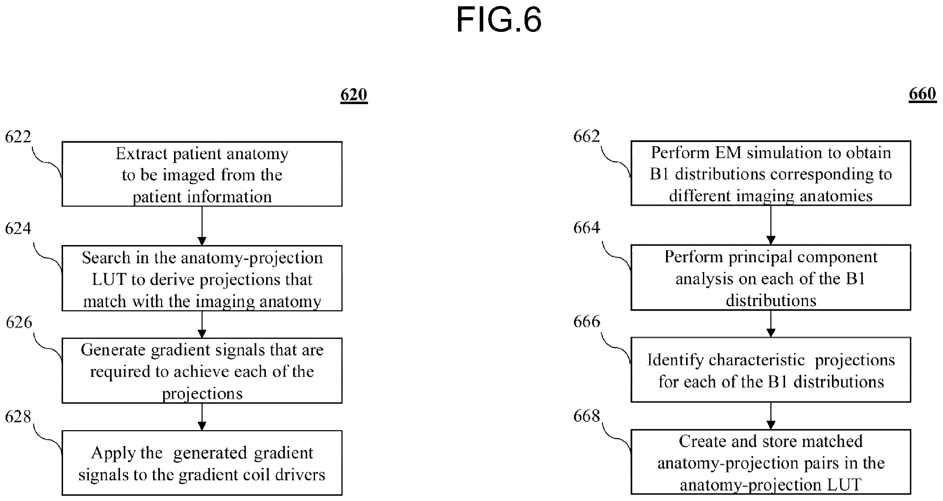

FIG. 6 shows a non-limiting example of a flow chart of a process of determining one or more projections along which projection data is acquired by the MRI scanner, according to one embodiment of the present disclosure. On the left side of FIG. 6 is a process 620 for determining the projections in accordance with the patient information received. At step 622, information regarding the imaging anatomy is extracted from the patient information. At step 624, a search is performed in the anatomy-projection look-up table 520 to extract one or more projections that match with the imaging anatomy. At step 626, gradient signals required to achieve each of the projections are generated. At step 628, the gradient signals are applied to the gradient coil drivers of the MRI scanner.

On the right side of FIG. 6 is a process 660 of offline preparing the anatomy-projection look-up table 520 used in the process 620. At step 662, electromagnetic (EM) simulations are performed to obtain B1 distributions corresponding to different imaging anatomies. At step 664, principal component analysis (PCA) is performed on each of the B1 distributions. At step 666, one or more characteristic projections are identified for each of the B1 distributions. At step 668, matched anatomy-projection pairs are created and stored in the anatomy-projection look-up table 520. Although electromagnetic simulations are used in FIG. 6 to prepare the anatomy-projection look-up table 520, one skilled in the art can appreciate that other means are possible. For example, data for creating the anatomy-projection pairs can be collected from other physical simulations, research experiments on phantoms or volunteers, and/or clinical procedures on patients.

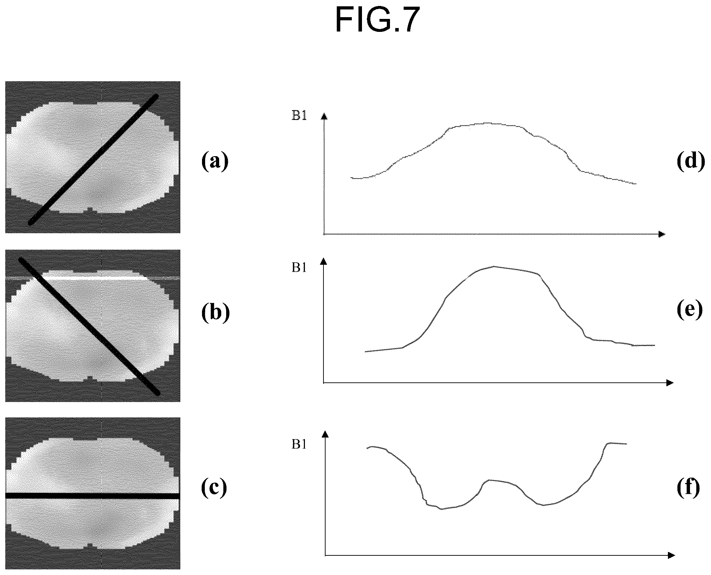

Portions (a)-(f) of FIG. 7 show a non-limiting example of different projections and B1 projection data acquired along the projections respectively, according to one embodiment of the present disclosure.

Typically, for B1 shimming, the goal is to solve for two fixed parameters, i.e., $\{A, \varphi\}$. This process is a lowresolution approach for a low degree-of-freedom solution. B1 projection data along a chosen orientation presents a characteristic signal in the projection data and contains enough spatially resolved information about the B1 pattern. When information about the scale of B1 and its spatial distribution is known, B1 shimming parameters $\{A, \varphi\}$ can be calculated using the B1 projection data.

In selecting shimming parameters, i.e., $\{A, \varphi\}$, various metrics or objectives can be considered. One natural objective is to obtain the shimming parameters which produce the least RF amplitude non-uniformity over a slice, as an integral over the slice. Another criterion could be to minimize the peak-to-peak variation over the slice. Yet other criteria are possible. Of particular interest may be the asymmetry of the variance in the B1 RF field. The human body has significant left-right symmetry, for example, in the brain. This symmetry can assist physicians in detecting various clinical conditions. A medical condition may cause some portion of brain, for example, to appear brighter or darker in an MRI image than the corresponding anatomical region on the contralateral side. Reducing other confounding technical sources of image asymmetry can make the task of diagnosis less complicated. Thus, even if overall image appearance is somewhat non-uniform due to RF transmit field non-uniformity, if that residual nonuniformity largely preserves left-right symmetry, the image may be preferred by clinicians.

In considering RF asymmetry, a single projection in the left-right direction may be judged in how close it comes to being symmetric. Or if two projections are disposed at different angular orientations, but if they correspond to a left-right mirrored pair of lines, then the asymmetry might be judged between the two projections, possibly flipping the values of the RF B1 values end-for-end along one of the two directions.

The selection of the projection orientations can be important to the resolving process. Typically, there exist principal axes for a B1 distribution in human subjects. Using methods such as principal component analysis (PCA), the optimal projection orientations can be identified.

PCA is an algorithm for sorting and comparing elements of variance in data. One exemplary use of PCA for choosing projection orientations is as follows. Collect an ensemble of B1+ map images, covering a range of human subjects, and covering a range of shimming parameter settings. Analysis proceeds, so as to emphasize the most significant variance in the population, and/or the aspects of variance which may most successfully modified by applying alternate shim value settings. Reformat the images to have similar centers, sizes, and intensity. Apply a common mask so as to mainly keep pixels which overlap in many of the images. Convert each image into a long 1-dimensional feature vector. Append the 1-dimensional vectors (row-wise or column-wise) into a single rectangular array. Apply the singular value decomposition algorithm (SVD), to identify singular vectors sorted in order of decreasing singular values. The SVD step is similar to an eigenvalue-eigenvector decomposition, but can be performed on rectangular arrays. Extract the highest few 1-dimensional singular vectors. For each, reformat it back from a 1-dimensional feature vector into a 2D image again. Each such resultant image is a principal component. Inspect the first few PCA images for radial directions which show the most overall variance, when the maps are projected along those radial readout directions. These are favorable and sensitive directions for acquiring shimming projections.

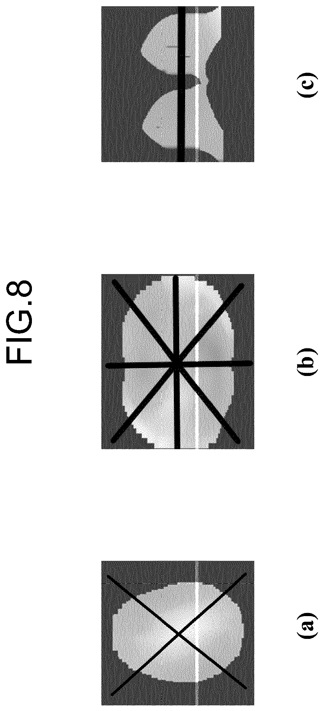

Portions (a)-(c) of FIG. 8 show a non-limiting example of different projections identified for different imaging anatomies. Each anatomy presents a characteristic B1 pattern. The number of projections can be specific for each imaging anatomy. A plurality of different models can be chosen to be included for each imaging anatomy, so as to cover "corner cases" (for example, metal implant, amputation/resection, etc.).

The orientation of the projections also can be specific for each imaging anatomy. The orientation can be chosen through PCA to align with the characteristic B1 pattern of the anatomy, for example. As illustrated in the portions (a)-(c) of FIG. 8, two orthogonal projections, oriented at about 45° can be chosen for the head (the portion (a) of FIG. 8). For the body (the portion (b) of FIG. 8), 2 orthogonal projections or 4 projections separated by about 45° can be chosen. Both breasts and thighs can use one horizontal projection due to their simple B1 patterns. Note that the specific angles shown in the portions (a)-(c) of FIG.8 are not restrictive. Other angular orientations are possible without departing from the scope of the present disclosure. For example, an angle of 40°, 60°, etc., can be used instead of 45° shown in the portion (a) of FIG. 8.

In one non-limiting embodiment, a minimum number of projections necessary for B1 shimming is determined to save scan time. Taking the head as an example, two projections can be determined. When the Bloch-Siegert Shift method is used, only four shots (two projections and two frequency offsets for each projection) are needed to obtain sufficient information. With this projection-based shimming approach, B1 shimming data can be acquired in less than one second. In an alternative embodiment, more projections can be acquired in order to add stability to the solution. As such, the orientation and number of the projections illustrated in the portions (a)-(c) of FIG. 8 is not limiting upon the present disclosure.

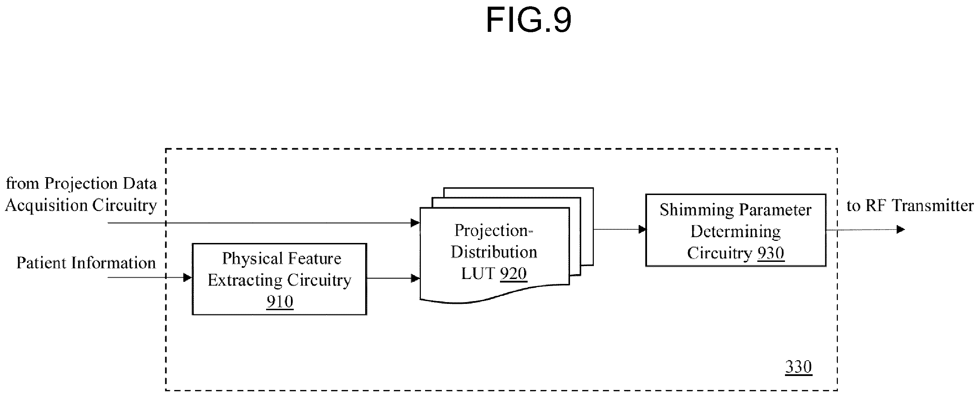

In the following, multiple approaches for solving a final output of {A, φ} from the B1 projection data will be described with reference to FIGs. 9-16.

In one non-limiting embodiment, translation from the B1 projection data to a final output of shimming parameters {A, φ} is conducted by a search within a dictionary or look-up table. It is observed that most patients share a characteristic B1 pattern. For example, it is known that axial slices of heads have a finite range of elliptical aspect ratios, e.g., from circle to ellipse. For a given size and aspect ratio, the patterns are very similar.

Thus, for each imaging anatomy, a group of B1 distributions can be pre-defined for different dimensional scales and aspect ratios, for example. More elements can be added to take into account features in more dimensions, such as body fat composition, male/female, age (e.g., child, youth, adult, senior), etc. Such a group of B1 distributions can be built offline by electromagnetic simulations using Sim4Life or a similar platform. Projection data of each of these B1 distributions can be generated, and a {A, φ} solution for each B1 distribution can be solved *a priori*. Thus, a look-up table can be prepared, each entry of which includes a B1 distribution and physical features, projection data, and a {A, φ} solution that are correlated to that B1 distribution. Then, a dictionary search (by brute force or other means) can be conducted in the look-up table to find a B1 distribution that has matched B1 projection data and matched physical features. For the matched B1 distribution, the optimized {A, φ} is already known.

FIG. 9 shows a non-limiting example of a block diagram of shimming parameter resolving circuitry that resolves a set of shimming parameters based on closely matching projection data acquired, according to one embodiment of the present disclosure. The shimming parameter resolving circuitry 330 includes physical feature extracting circuitry 910, a projection-distribution look-up table 920, and shimming parameter determining circuitry 930. The physical feature extracting circuitry 910 extracts one or more physical features from the patient information. The physical features can be a dimensional scale and/or an aspect ratio, for example. Based on the projection data received from the projection data acquisition circuitry 320 and the extracted physical features, a matched B1 distribution is searched out in the projection-distribution look-up table 920. Based on the matched B1 distribution, the shimming parameter determining circuitry 930 determines a set of shimming parameters and applies them to the RF transmitter.

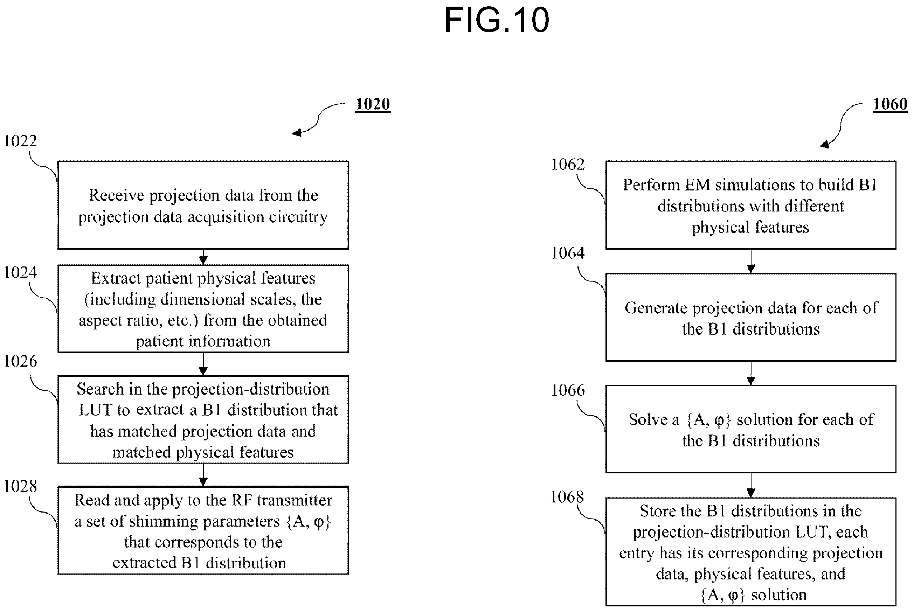

FIG. 10 shows a non-limiting example of a flow chart of a process that resolves a set of shimming parameters based on projection data acquired, according to one embodiment of the present disclosure. On the left of FIG. 10 is a process 1020 for resolving the shimming parameters using the projection-distribution look-up table 920. At step 1022, the projection data is received from the projection data acquisition circuitry 320. At step 1024, one or more physical features (e.g., a dimensional scale and/or an aspect ratio, etc.) are extracted from the patient information. At step 1026, a search is performed in the pre-stored projection-distribution look-up table 920 to extract a B1 distribution that has matched projection data and matched physical features. At step 1028, a set of shimming parameters that are correlated with the extracted B1 distribution are read and applied to the RF transmitter of the MRI scanner.

On the right side of FIG. 10 is a process 1060 for offline preparation of the projection-distribution look-up table 920. At step 1062, electromagnetic simulations are performed to build B1 distributions for different physical features (including, but not limited to, a dimensional scale and/or an aspect ratio, etc.). At step 1064, projection data is generated for each of the B1 distributions. At step 1066, a shimming parameter solution is solved for each of the B1 distributions. At step 1068, the B1 distributions are stored in the projection-distribution look-up table 920. Each entry has a B1 distribution and projection data, physical features, and a shimming parameter solution that are correlated to the B1 distribution. Although electromagnetic simulations are used in FIG. 10 to prepare the projection-distribution look-up table 920, one skilled in the art can appreciate other means are possible. For example, data for creating the distribution entries can be collected from other physical simulations, research experiments on phantoms or volunteers, and/or clinical procedures on patients.

In an alternative embodiment, machine learning is used to map one set of information into another domain. In this case, physical features and the measured spatial B1 projection data are inputted into a neural network. The output of the neural network is the optimal shimming parameter set {A, φ}. Instead of looking up the {A, φ} solution in a pre-defined dictionary or look-up table, the machine learning approach learns the mapping with the neural network and supervised learning. The network learns the transfer function in advance from training data including B1 distributions, physical features, projection data, and shimming parameter sets {A, φ}. As it is computationally faster to execute a machine learning inference than a dictionary search, this approach gives an additional advantage in speed.

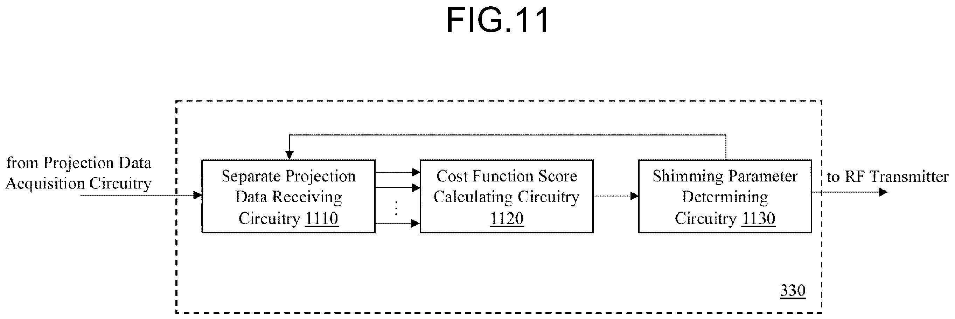

FIG. 11 shows a non-limiting example of a block diagram of shimming parameter resolving circuitry that resolves a set of shimming parameters based on projection data acquired, according to one embodiment of the present disclosure. The shimming parameter resolving circuitry 330 includes separate projection data receiving circuitry 1110, cost function score calculating circuitry 1120, and shimming parameter determining circuitry 1130. The shimming parameter determining circuitry 1130 selects a plurality of different shimming parameter sets applied to the RF transmitter. The separate projection data receiving circuitry 1110 obtains projection data from the projection data acquisition circuitry 320, and outputs to the cost function score calculating circuitry 1120 separate projection data acquired along one or more orientations, with the different shimming parameter sets applied. The cost function score calculating circuitry 1120 calculates a cost function score with respect to each of the plurality of different shimming parameter sets. Based on the cost function scores, the shimming parameter determining circuitry 1130 identifies a set of shimming parameters that has the lowest cost function score, and outputs the set of shimming parameters to the RF transmitter of the MRI scanner.

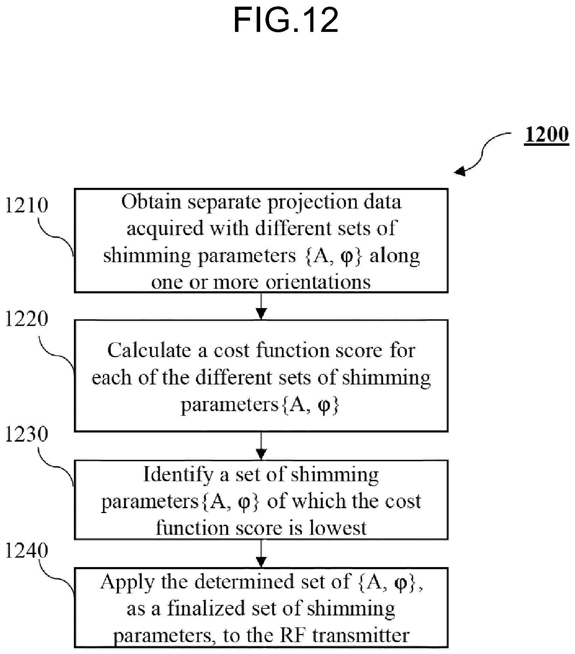

FIG. 12 shows a non-limiting example of a flow chart of a process 1200 that resolves a set of shimming parameters based on projection data acquired, according to one embodiment of the present disclosure. At step 1210, with respect to different sets of shimming parameters, projection data is received along one or more orientations. For example, projection data can be obtained along two orientations (e.g., about 45° and about 135°, about 0° and about 90°, etc.), for different sets of shimming parameters (for example, one parameter set can be {A=0dB, φ=50°} and another can be {A=3dB, φ=120°}). At step 1220, a cost function score is calculated for each of the different sets of shimming parameters. As described below, the cost function score can be based on an L1-norm or an L2-norm, for example, and cost function scores in other forms are possible as well. At step 1230, based on the cost function scores calculated in step 1220, a set of shimming parameters is identified that has the lowest cost function score. At step 1240, the identified set of shimming parameters is determined as the finalized set of shimming parameters and applied to the RF transmitter.

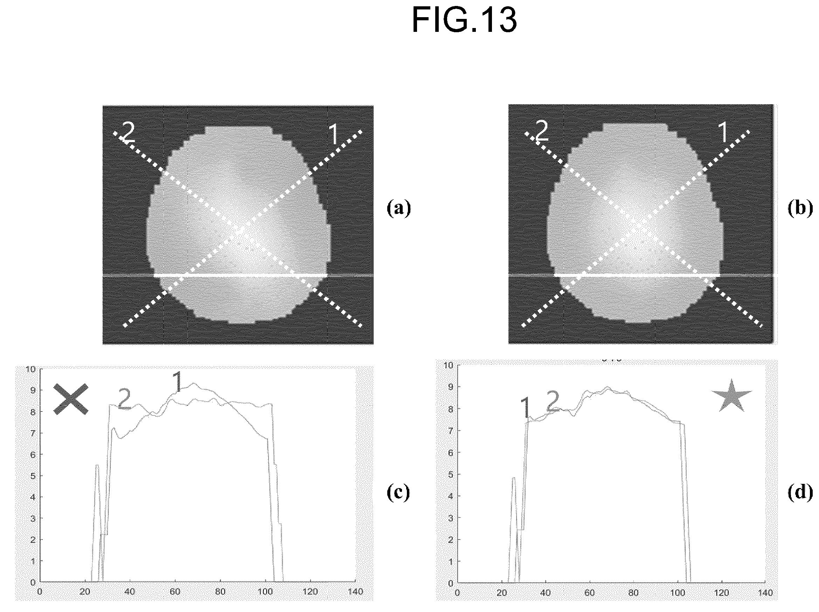

Portions (a)-(d) of FIG. 13 show a non-limiting example of acquiring projection profiles along two or more orientations, with respect to different sets of shimming parameters, according to one embodiment of the present disclosure. The portion (a) of FIG. 13 shows two exemplary orientations, labeled with 1 and 2, with a first set of shimming parameters applied to the RF transmitter. Projection data profiles generated along the two orientations 1 and 2 are shown in the portion (c) of FIG. 13. Similarly, the portion (b) of FIG. 13 shows two orientations 1 and 2 with a second set of shimming parameters applied, and the corresponding projection data profiles acquired are shown in the portion (d) of FIG. 13. It is appreciated that the numbers of the sets of shimming parameters and the orientations are not restrictive. Without departing from the scope of the disclosure, other numbers of shimming parameter sets and orientations are feasible.

According to Equation (1) below, a cost function score is calculated for each set of shimming parameters, so as to measure the difference between the projection data profiles acquired along orientations 1 and 2:

$$\text{Cost function score} = \frac{\sum_{x=1}^{x=Nx}(|B1_1(x) - B1_2(x)|)^L}{Nx} \qquad (1)$$

wherein L= 1 or 2, x denotes an index of pixels along the projection data profile, and Nx refers to the number of all of those pixels. Alternatively, Nx refers to the number of the pixels within a certain summation range. For example, the summation range can be a range for which there is good data, a range for which the signal magnitude is higher than some threshold or some noise floor, or a range that is windowed or cropped to exclude potentially poorer quality data at the ends of the profile curve (e.g., keeping the central 80% of the data while discarding 10% of the values on either end), etc. Given L=1, an L1-norm-based score is obtained from the calculation of Equation (1). The L1-norm is the sum of absolute values of the difference between the projection data profiles along the orientations 1 and 2.

Here, the L1-norm-based score is effectively a measure of symmetry of the B1 field. As the projection profiles shown in the portion (d) of FIG. 13 have a smaller difference between each other than that between the two projection profiles shown in the portion (c) of FIG. 13, the L1-norm-based score calculated for the second set of shimming parameters is smaller than that for the first set of shimming parameters. The smaller cost function score reveals that the second set of shimming parameters is better as it leads to a more uniform B1 field distribution. Therefore, by comparing cost function scores calculated for a plurality of different sets of shimming parameters, a best set of shimming parameters can be identified.

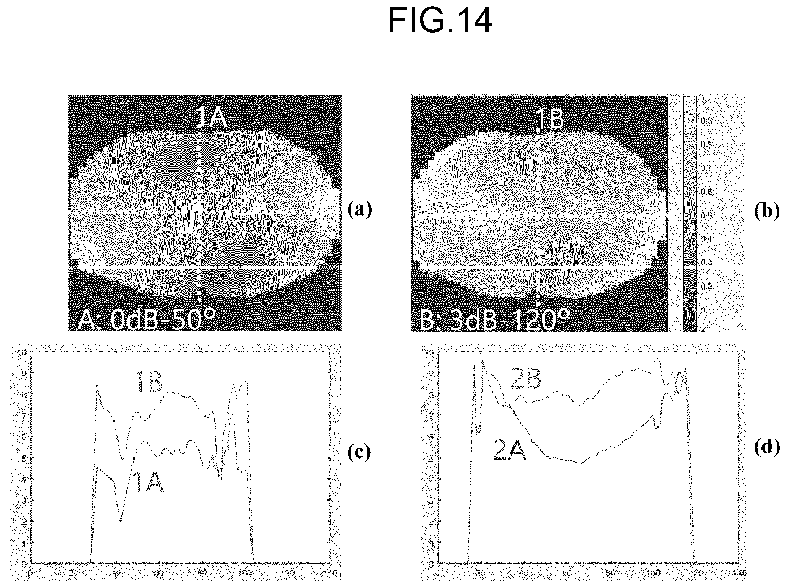

It is possible to evaluate a cost function score in other forms than the L1-norm-based score to identify a best set of shimming parameters. Portions (a)-(d) of FIG. 14 show a non-limiting example of acquiring projection profiles along one or more orientations, with respect to different sets of shimming parameters, according to one embodiment of the present disclosure. The portion (a) of FIG. 14 shows two exemplary orientations, labeled with 1A and 2A, with a first set of shimming parameters (e.g., A=0dB; φ=50°) applied to the RF transmitter. The corresponding projection data profiles 1A and 2A obtained along the two orientations are shown in the portions (c) and (d) of FIG. 14, respectively. Similarly, the portion (b) of FIG. 14 shows two orientations 1B and 2B with a second set of shimming parameters (e.g., A=3dB; φ=120°) applied, and the corresponding projection data profiles 1B and 2B acquired are shown in the portions (c) and (d) of FIG. 14.

For each set of shimming parameters, an L2-norm-based cost function score can be calculated according to Equation (2) below:

$$\text{Cost function score} = \frac{\sum_{x=1}^{x=Nx}\left(|B1_1(x)-B1_{target}|\right)^2}{Nx} \qquad (2)$$

wherein x and Nx have similar meanings as those in Equation (1), and $B1_{target}$ refers to a preferred B1 projection data profile that can be obtained with any feasible means. The cost function score in this embodiment is based on an L2-norm, which represents the sum of squared differences between the projection data profile along an orientation and fixed, target B1 field data. The projection data profile obtained along either one or both of the two orientations shown in the portions (a) and (b) of FIG. 14 can be used in calculation of this L2-norm-based score for the two different sets of shimming parameters. Again, by comparing cost function scores calculated for a plurality of different sets of shimming parameters, a best set of shimming parameters can be identified.

With the approach shown in FIGs. 11 and 12, it is possible to select two projections (e.g., shown in the portions (a)-(d) of FIG. 13) or even only one projection (e.g., shown in the portions (a)-(d) of FIG 14) to optimize the B1 shimming. Alternatively, more projections can be added to improve the robustness of the solution.

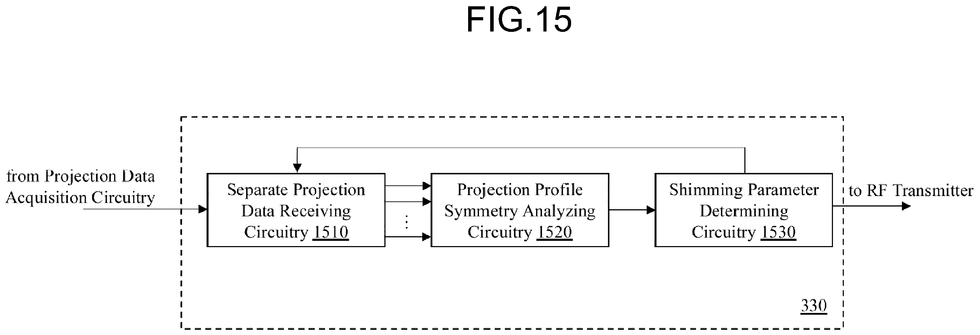

FIG. 15 shows a non-limiting example of a block diagram of shimming parameter resolving circuitry that resolves a set of shimming parameters based on projection data acquired, according to one embodiment of the present disclosure. The shimming parameter resolving circuitry 330 includes separate projection data receiving circuitry 1510, projection profile symmetry analyzing circuitry 1520, and shimming parameter determining circuitry 1530. The shimming parameter determining circuitry 1530 generates shimming parameters to be applied to the RF transmitter. In particular, by applying appropriate shimming parameters, the shimming parameter determining circuitry 1530 can switch one of the transmit channels ON, and the other channels OFF. The separate projection data receiving circuitry 1510 receives the projection data acquired with different transmit channels switched ON. The projection profile symmetry analyzing circuitry 1520 evaluates the effect of different transmit channels on the symmetry of the projection profile. Based on the result of the evaluation, the shimming parameter determining circuitry 1530 determines a finalized set of shimming parameters that maximizes the symmetry of the projection profile.

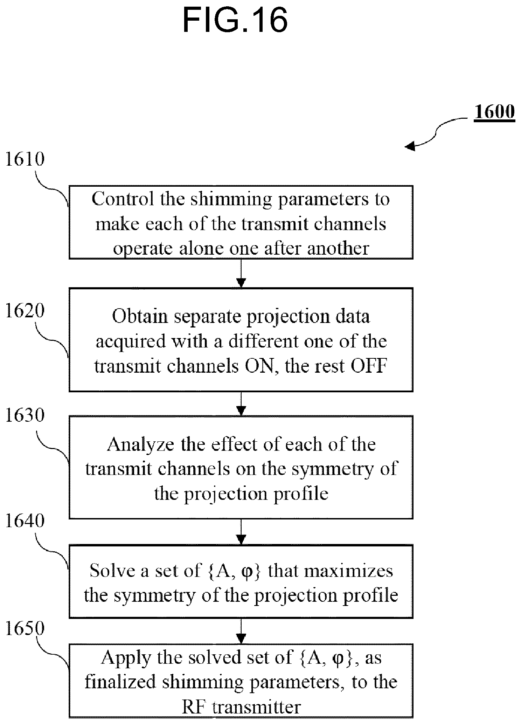

FIG. 16 shows a non-limiting example of a flow chart of a process 1600 that resolves a set of shimming parameters based on projection data acquired, according to one embodiment of the present disclosure. The process starts at step 1610, in which the shimming parameters applied to the RF transmitter are controlled to make each of the transmit channels operate alone. At step 1620, projection data is received from the projection data acquisition circuitry 320. The separate projection data is acquired with a different one of the transmit channels turned ON, the rest OFF. At step 1630, the effect of each of the transmit channels on the symmetry of the projection profile is analyzed. At step 1640, a set of shimming parameters {A, φ} is solved. Such a set of shimming parameters maximizes the symmetry of the projection profile. At step 1650, the solved set of shimming parameters {A, φ} is applied, as a finalized set of shimming parameters, to the RF transmitter.

Similar to the approach shown in FIGs. 11 and 12, this approach does not need a pre-calculated look-up table or a trained neural network. Instead, this approach uses the symmetry of the projection profile (or the cost function score calculated for each set of shimming parameters, for the approach shown in FIGs. 11 and 12) as a surrogate measure of uniformity of the B1 field distribution. By evaluating the effect of each transmit channel on the symmetry of the B1 data in the profile, a best set of {A, φ} can be solved to maximize the symmetry.

Preferably, the orientation of the projection is selected along the characteristic directions of the B1 field distribution. More preferably, the orientation of the projection is selected along the characteristic orientation displaying the most asymmetry, so as to add sensitivity to the approach. With this approach, it is possible to select only one projection. Alternatively, more projections can be added to improve the robustness of the solution.

Note that for the approaches illustrated in FIGs 15, and 16, the choice of projection orientation can be important. If 'hot' and 'cold' spots (+/- regions) in the B1 field distribution overlap, it will be difficult to differentiate the effect of B1 shimming. Taking the projections shown in the portions (a)-(c) of FIG. 7 as an example, the portion (a) of FIG. 7 illustrates a good choice because +/- regions are mostly not overlapped in the projection, i.e., when the B1 amplitudes are combined along a ray perpendicular to the readout direction of the projection. This combination task of forming projections is similar to taking a mean value; the amplitude of the projected data does not increase simply based upon the length of the ray through the subject. Also, the portion (b) of FIG. 7 is a good choice because +/regions are mostly not overlapped in the projection.

However, the portion (c) of FIG. 7 is not a good choice because +/regions overlap more than the portions (a) and (b) of FIG. 7.

For all embodiments described above, any B1 measuring method can be used, including but not limited to, the Double Angle Method (DAM), Actual Flip angle Imaging (AFI), Dual Refocusing Echo Acquisition Mode (DREAM), Phase Sensitive (PS), Saturation Recovery, and the Bloch-Siegert Shift method, for example. The approaches given in this disclosure will perform best with a B1 measuring method immune to tissue composition and relaxation biases. If the B1 data is contaminated by different tissue types, the projection will have an error, because it is effectively an integration of all tissue along the projection. Thus, the Bloch-Siegert Shift method is preferable because it is generally largely unaffected by a wide range of tissue compositions.

Preferably, a region-of-interest within the projection can be chosen. For example, only the central 80% of the projection is considered within the solution so as to avoid subcutaneous fat. Conventional Cartesian B1 mapping methods normally would allow support using a region-of-interest centered anywhere in the volume. Any shape, size, and position of the region-of-interest is possible with a fully encoded map. In contrast, the projection-based approaches described in this disclosure are generally limited in flexibility of selecting region-of-interest locations due to the nature of a projection. B1 intensity information along a perpendicular line is intrinsically combined in the basic one-dimensional readout. Unless further features are implemented in the shim acquisition by projections, there would be no method to collect the data only along some finite subsegment of the perpendicular ray. In other words, data in the spatial dimension orthogonal to the projection readout dimension is effectively averaged when measuring the projection. Therefore, the position in this orthogonal dimension will not be resolved, and thus, the boundaries of the region-of-interest in that dimension will not be resolved.

Further, a portion of the volume can be selected. For example, the inner-volume method with RF slice selection along different gradient axes (e.g., 2D spatial RF selection or "pencil beam" method) can be used. The pencil beam approach (sometimes called "inner volume selection") uses two or more RF slice-selective pulses with fully- or partiallyorthogonal RF selection gradient directions to invoke an MR signal from only the intersecting geometric regions selected by the RF pulses. It is an effective way to generate a signal only along a rectangular column, after which the readout direction can be applied along the length of the column.

In addition, more than one projection per repetition time (TR) (a "bowtie" method) can be acquired using multiple readouts per excitation. For the "bowtie" readout method, following the RF excitation, the RF pulses and pattern of gradient waveforms in the pulse sequence are designed in such a way as to measure two or more echo signals along different spatial trajectories. Specifically, after the excitation of a slice, a conventional readout may collect data in the k-space along one line at some angle through the center of the k-space. Once that line of data is collected, an extra gradient lobe in some other direction can move the net k-space encoding to an extreme location at some other angular direction relative to the center of the k-space. Then another readout gradient and data sampling can trace back along that second angular direction, through the center of the k-space, and to a geometrically opposite position in the k-space. This configuration, with two or more distinct angular readout directions each intersecting at the center, can be called a "bowtie readout method."

Compared to conventional Cartesian B1 maps, a much shorter pre-scan time can be achieved with the approaches described in this disclosure. If the Bloch-Siegert Shift method is used and the repetition time (TR) is 200ms, it takes 0.4 seconds for B1 shimming for breasts or thighs (1 projection $\times$ 2 frequencies), 0.8 seconds for the head (2 projections $\times$ 2 frequencies), and 1.6 seconds for the body (4 projections $\times$ 2 frequencies). Therefore, B1 projection data can be acquired in approximately 1 second, compared to about 26 seconds (32 encodes $\times$ 2 frequencies $\times$ 2 channels, if a separate map is measured for each channel) for even the lowest resolution Cartesian B1 map. Moreover, it is inexpensive to select more projections to add more stability to the solution, if necessary. The added costs are on the order of about one more second.

Furthermore, under the same principle as free-breathing radial acquisition, it is expected that breath hold is not necessary for the approaches described in the present disclosure, because they measure simple projections through a center of a volume each time. Also, in general, as the scanning duration of the shimming acquisition projections is greatly reduced, there is a less likely chance of data being inconsistent or corrupted by patient motion. Bulk patient motion in a window of about one second is routinely less significant than the total motion encountered across some tens of seconds.

Although the translation of B1 projection data to the {A, φ} solution requires some computation time, both the look-up-table approach and the machine-learning approach take a limited duration to calculate the {A, φ} solution. This computation time can be hidden during other pre-scans (e.g., a coil map). Traditional B1 shimming solutions require computation time as well to solve individual channels of B1 map input.

In general, the approaches described in this disclosure provide significant scan time savings compared with the conventional methods, and there is no need to acquire a complete B1 map in order to calculate simple two-parameter settings for B1 shimming. By measuring a few projections along characteristic orientations, sufficient information can be obtained from the projection data to determine B1 shimming parameters {A, φ}.

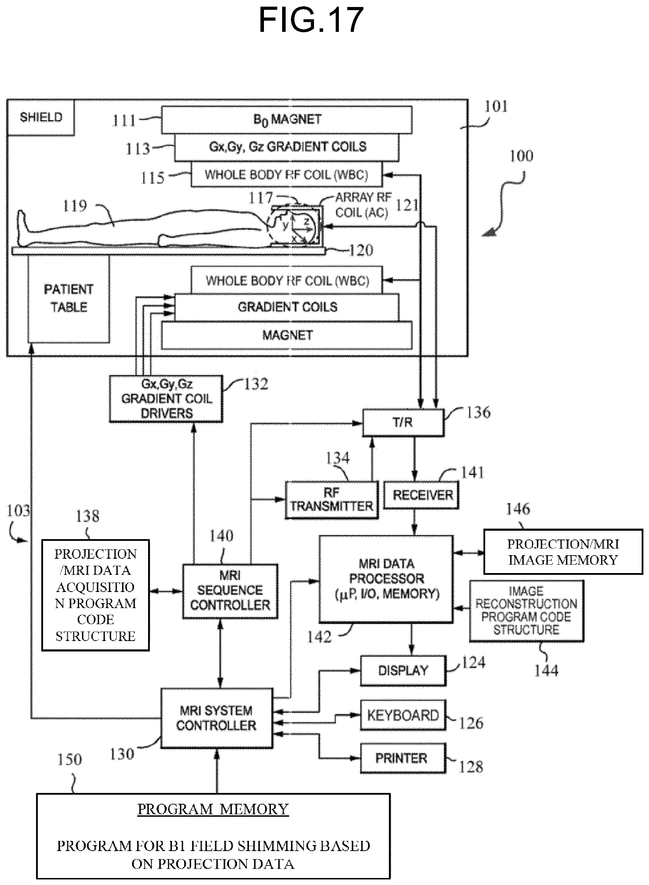

Referring now to FIG. 17, a non-limiting example of a magnetic resonance imaging (MRI) system 100 is shown. The MRI system 100 depicted in FIG. 17 includes a gantry 101 (shown in a schematic cross-section) and various related MRI system components 103 interfaced therewith. At least the gantry 101 is typically located in a shielded room. The MRI system 100 geometry depicted in FIG. 17 includes a substantially coaxial cylindrical arrangement of the static field B0 magnet 111, a Gx, Gy, and Gz gradient coil set 113, and a large whole-body RF coil (WBC) 115. Along a horizontal axis of this cylindrical array of elements is an imaging volume 117 shown as substantially encompassing the head of a patient 119 supported by a patient table 120.

One or more smaller array RF coils 121 can be more closely coupled to the patient's head (referred to herein, for example, as "scanned object" or "object") in imaging volume 117. As those in the art will appreciate, compared to the WBC (whole-body coil), relatively small coils and/or arrays, such as surface coils or the like, are often customized for particular body parts (e.g., arms, shoulders, elbows, wrists, knees, legs, chest, spine, etc.). Such smaller RF coils are referred to herein as array coils (AC) or phased-array coils (PAC). These can include at least one coil configured to transmit RF signals into the imaging volume 117, and a plurality of receiver coils configured to receive RF signals from an object, such as the patient's head, in the imaging volume 117.

The MRI system 100 includes an MRI system controller 130 that has input/output ports connected to a display 124, a keyboard 126, and a printer 128. As will be appreciated, the display 124 can be of the touch-screen variety so that it provides control inputs as well. A mouse or other I/O device(s) can also be provided.

The MRI system controller 130 interfaces with an MRI sequence controller 140, which, in turn, controls the Gx, Gy, and Gz gradient coil drivers 132, as well as the RF transmitter 134, and the transmit/receive switch 136 (if the same RF coil is used for both transmission and reception). The RF transmitter 134 may be composed of two or more transmitter channels for driving two or more RF transmit coils or ports on coils, as is used for RF shimming. The MRI sequence controller 140 includes suitable program code structure 138 for implementing MRI imaging (also known as nuclear magnetic resonance, or NMR, imaging) techniques including B1 field shimming. MRI sequence controller 140 can be configured for MR imaging with or without parallel imaging. Moreover, the MRI sequence controller 140 can facilitate one or more preparation scan (pre-scan) sequences, and a scan sequence to obtain a main scan magnetic resonance (MR) image (referred to as a diagnostic image). MR data from pre-scans can be used, for example, to determine shimming parameters for the whole-body RF coil 115 and/or the array RF coils 121.

The MRI system components 103 include an RF receiver 141 providing input to MRI data processor 142 so as to create processed image data, which is sent to display 124. The MRI data processor 142 is also configured to access previously generated MR data, images, and/or projection data, such as, for example, projection data acquired with different perturbance in shimming parameters, projection data acquired with different transmit channels, and/or system configuration parameters 146, and program code structures 144 and 150.

In one embodiment, the MRI data processor 142 includes processing circuitry. The processing circuitry can include devices such as an application-specific integrated circuit (ASIC), configurable logic devices (e.g., simple programmable logic devices (SPLDs), complex programmable logic devices (CPLDs), and field programmable gate arrays (FPGAs), and other circuit components that are arranged to perform the functions recited in the present disclosure, such as described with respect to FIGs. 3, 5, 9, 11, and 15.

The MRI data processor 142 executes one or more sequences of one or more instructions contained in the program code structures 144 and 150. Alternatively, the instructions can be read from another computer-readable medium, such as a hard disk or a removable media drive. One or more processors in a multi-processing arrangement can also be employed to execute the sequences of instructions contained in the program code structures 144 and 150. In alternative embodiments, hard-wired circuitry can be used in place of or in combination with software instructions. Thus, the disclosed embodiments are not limited to any specific combination of hardware circuitry and software. For example, the program code structure 150 can store instructions that when executed perform method 400.

Additionally, the term "computer-readable medium" as used herein refers to any non-transitory medium that participates in providing instructions to the MRI data processor 142 for execution. A computer readable medium can take many forms, including but not limited to, non-volatile media or volatile media. Non-volatile media includes, for example, optical, magnetic disks, and magneto-optical disks, or a removable media drive. Volatile media includes dynamic memory.

Also illustrated in FIG. 17 is a generalized depiction of an MRI system program storage (memory) 150, where stored program code structures such as instructions to perform method 400 are stored in non-transitory computer-readable storage media accessible to the various data processing components of the MRI system 100. As those in the art will appreciate, the MRI system program storage 150 can be segmented and directly connected, at least in part, to different ones of the MRI system components 103 processing computers having most immediate need for such stored program code structures in their normal operation (i.e., rather than being commonly stored and connected directly to the MRI system controller 130).

In addition to performing the steps (including, for example, the steps of any of methods 400, 620, 1020, 1200, 1600) for determining and applying the shimming parameters for an individual patient, the MRI system 100 may, in response to the execution of instructions by MRI data processor 142 also perform the steps (for example, the steps of any of methods 660, 1060) for preparing the look-up tables or training the neural network. Alternatively, at least some of the processing steps for preparing the look-up tables and training the neural network may be performed by at least one processor of a separate data processing system.

Additionally, the MRI system 100 as depicted in FIG. 17 can be utilized to practice exemplary embodiments described herein above. The MRI system components 103 can be divided into different logical collections of "boxes" and typically comprise numerous digital signal processors (DSP), microprocessors and special purpose processing circuits (e.g., for fast A/D (Analog to Digital) conversions, fast Fourier transforming, array processing, etc.). Each of those processors is typically a clocked "state machine" wherein the physical data processing circuits progress from one physical state to another upon the occurrence of each clock cycle (or predetermined number of clock cycles).

Furthermore, not only does the physical state of the processing circuits (e.g., CPUs, registers, buffers, arithmetic units, etc.) progressively change from one clock cycle to another during the course of operation, the physical state of associated data storage media (e.g., bit storage sites in magnetic storage media) is transformed from one state to another during operation of such a system. For example, at the conclusion of an image reconstruction process and/or sometimes an image reconstruction map (e.g., coil sensitivity map, unfolding map, ghosting map, a distortion map etc.) generation process, an array of computer-readable accessible data value storage sites in physical storage media will be transformed from some prior state to a new state wherein the physical states at the physical sites of such an array vary between minimum and maximum values to represent real world physical events and conditions. As those in the art will appreciate, such arrays of stored data values represent and also constitute a physical structure, as does a particular structure of computer control program codes that, when sequentially loaded into instruction registers and executed by one or more CPUs of the MRI system 100, causes a particular sequence of operational states to occur and be transitioned through within the MRI system 100.

Numerous modifications and variations are possible in light of the above teachings. As such, the foregoing descriptions of embodiments of the disclosure are not intended to be limiting. Rather, any limitations to embodiments of the disclosure are presented in the following claims.

According to at least one aspect of the embodiments described above, it is possible to save the scan time of pre-scan.

**Claims**

1.  A shimming method (400) for performing patient-specific B1 field shimming in a magnetic resonance imaging system, comprising:

    obtaining (410) patient information of a patient to be imaged by the magnetic resonance imaging system;
    determining (420) an orientation of a projection based on the obtained patient information;
    acquiring (430) B1 projection data, using the magnetic resonance imaging system, along the determined orientation of the projection;
    determining (440) a set of B1 shimming parameters based on the acquired B1 projection data;
    controlling (450) the magnetic resonance imaging system based on the determined set of B1 shimming parameters.

2.  The shimming method (400) of claim 1 , wherein the step of determining (420) the orientation of the projection further comprises:

    extracting (622), from the patient information, a specific imaging anatomy, and
    obtaining (624), from a first look-up table, using the extracted imaging anatomy as a key, the orientation of the projection, and

    the step of acquiring (430) the B1 projection data further comprises:

    generating (626) gradient signals based on the obtained orientation of the projection, and
    applying (628) the generated gradient signals to gradient coil drivers of the magnetic resonance imaging system.

3.  The shimming method (400) of claim 2, further comprising:

    determining (662) B1 field distributions corresponding to different particular imaging anatomies, respectively;
    performing (664) principal component analysis on each of the determined B1 field distributions;
    identifying (666), based on a result of the principal component analysis, a characteristic projection for each of the determined B1 field distributions; and
    storing (668) the identified characteristic projections and the corresponding imaging anatomies as matched pairs in the first look-up table.

4. The shimming method (400) of claim 3, wherein the step of determining (662) the B1 field distributions further comprises determining the B1 field distributions based on data collected in a physics simulation, a phantom experiment, an in vivo experiment, and/or a clinical procedure conducted on a patient.

5. The shimming method (400) of any of claims 2 to 4, wherein the step of determining (440) the set of B1 shimming parameters further comprises:

   extracting (1024), from the patient information, a specific physical feature;
   obtaining (1026), from a second look-up table, a B1 field distribution, wherein a particular physical feature related to the B1 field distribution matches the extracted physical feature, and particular projection data related to the B1 field distribution matches the acquired projection data; and
   determining (1026) a particular set of B1 shimming parameters related to the obtained B1 field distribution to be the determined set of B1 shimming parameters.

6. The shimming method (400) of claim 5, further comprising:

   determining (1062) B1 field distributions for different corresponding physical features, respectively;
   generating (1064) corresponding projection data for each of the determined B1 field distributions;
   determining (1066) a corresponding set of B1 shimming parameters for each of the determined B1 field distributions; and
   storing (1068) the determined B1 field distributions in the second look-up table, wherein each of the determined B1 field distributions is stored in association with the corresponding physical feature, the corresponding projection data, and the corresponding set of B1 shimming parameters.

7. The method (400) of claim 5 or claim 6, wherein the extracted physical feature includes at least one of a dimensional scale of the imaging anatomy, an aspect ratio of the imaging anatomy, a body fat composition of the patient, a gender of the patient, and an age of the patient.

8. The shimming method (400) of any of claims 2 to 4, wherein
   the step of determining the set of B1 shimming parameters further comprises:

   extracting, from the patient information, a specific physical feature;
   applying the extracted physical feature and the acquired projection data to a trained neural network; and
   determining the set of B1 shimming parameters from outputs of the neural network.

9. The shimming method (400) of any of claims 1 to 4, wherein
   the step of determining (440) the set of B1 shimming parameters further comprises:

   receiving (1210), along the determined orientation of the projection, B1 projection data corresponding to each of a plurality of sets of B1 shimming parameters, respectively;
   calculating (1220), based on the received B1 projection data, a cost function score corresponding to each of the plurality of sets of B1 shimming parameters, respectively;
   identifying (1230) a particular set of B1 shimming parameters which corresponds to a lowest cost function score; and
   determining (1240) the identified particular set of B1 shimming parameters to be the determined set of B1 shimming parameters.

10. The shimming method (400) of any of claims 1 to 4, wherein the step of determining (440) the set of B1 shimming parameters further comprises:

    controlling (1610) a set of B1 shimming parameters to switch ON each of individual transmit channels in an RF transmitter of the magnetic resonance imaging system, with other transmit channels switched OFF;
    receiving (1620) particular B1 projection data acquired with each of the individual transmit channels switched ON, respectively;
    analyzing (1630) the received particular B1 projection data to evaluate an effect of each of the individual transmit channels on a symmetry of a profile of the B1 projection data;

calculating (1640) a particular set of B1 shimming parameters that maximizes the symmetry of the profile of the B1 projection data; and
determining (1640) the calculated particular set of B1 shimming parameters to be the determined set of B1 shimming parameters.

11. The shimming method (400) of claim 10, wherein the step of determining (420) the orientation of the projection further comprises determining the orientation such that the profile of the B1 projection data acquired along the determined orientation has minimum symmetry.

12. The shimming method (400) of any preceding claim, wherein the step of acquiring (430) the B1 projection data further comprises at least one of:

acquiring the projection data using a Bloch-Siegert Shift method, a Double Angle method, an Actual Flip Angle method, a Dual Refocusing Echo Acquisition Mode method, a Phase Sensitive method, or a Saturation Recovery method; and
performing 2D spatial selection to select a portion within a volume of the patient along the determined orientation of the projection.

13. The shimming method (400) of any preceding claim, wherein the step of acquiring (430) the B1 projection data further comprises performing more than one readout per excitation to acquire more than one projection per repetition time.

14. A shimming apparatus (300) for performing patient-specific B1 field shimming in a magnetic resonance imaging system, the apparatus comprising:
processing circuitry (310, 320, 330) configured to

obtain patient information of a patient to be imaged by the magnetic resonance imaging system;
determine an orientation of a projection based on the obtained patient information;
acquire B1 projection data, using the magnetic resonance imaging system, along the determined orientation of the projection; and
determine a set of B1 shimming parameters based on the acquired B1 projection data; control the magnetic resonance imaging system based on the determined set of B1 shimming parameters.

**Patentansprüche**

1. Shimverfahren (400) zur Durchführung eines patientenspezifischen B1-Feld-Shims in einem Magnetresonanztomographiesystem, umfassend:

Erhalten (410) von Patienteninformationen eines Patienten, der mit dem Magnetresonanztomographiesystem untersucht werden soll;
Bestimmen (420) einer Ausrichtung einer Projektion auf der Grundlage der erhaltenen Patienteninformationen;
Erfassen (430) von B1-Projektionsdaten unter Verwendung des Magnetresonanztomographiesysytems entlang der bestimmten Projektionsausrichtung;
Bestimmen (440) eines Satzes von B1-Shimparametern auf der Grundlage der erfassten B1-Projektionsdaten;
Steuern (450) des Magnetresonanztomographiesystems auf der Grundlage des bestimmten Satzes von B1-Shimparametern.

2. Shimverfahren (400) nach Anspruch 1, wobei der Schritt des Bestimmens (420) der Ausrichtung der Projektion weiter umfasst:

Extrahieren (622) einer spezifischen Bildgebungsanatomie aus den Patienteninformationen und
Erhalten (624) der Ausrichtung der Projektion aus einer ersten Nachschlagetabelle unter Verwendung der extrahierten Bildgebungsanatomie als Schlüssel, und
der Schritt des Erfassens (430) der B1-Projektionsdaten weiter umfasst:

Erzeugen (626) von Gradientensignalen auf der Grundlage der erhaltenen Ausrichtung der Projektion, und
Anwenden (628) der erzeugten Gradientensignale auf Gradientenspulentreiber des Magnetresonanztomographiesystems.

3. Shimverfahren (400) nach Anspruch 2, weiter umfassend:

Bestimmen (662) von B1-Feldverteilungen, die jeweils verschiedenen spezifischen Bildgebungsanatomien entsprechen;
Durchführen (664) einer Hauptkomponentenanalyse an jeder der bestimmten B1-Feldverteilungen;
Identifizieren (666) einer charakteristischen Projektion für jede der bestimmten B1-Feldverteilungen auf der Grundlage eines Ergebnisses der Hauptkomponentenanalyse; und
Speichern (668) der identifizierten charakteristischen Projektionen und der entsprechenden Bildgebungsanatomien als zusammengehörige Paare in der ersten Nachschlagetabelle.

4. Shimverfahren (400) nach Anspruch 3, wobei der Schritt des Bestimmens (662) der B1-Feldverteilungen weiter das Bestimmen der B1-Feldverteilungen auf der Grundlage von Daten umfasst, die in einer Physiksimulation, einem Phantomexperiment, einem Invivo-Experiment und/oder einem klinischen Eingriff, der an einem Patienten vorgenommen wurde, gesammelt wurden.

5. Shimverfahren (400) nach einem der Ansprüche 2 bis 4, wobei der Schritt des Bestimmens (440) des Satzes von B1-Shimparametern weiter umfasst:

Extrahieren (1024) eines spezifischen physischen Merkmals aus den Patienteninformationen;
Erhalten (1026) einer B1-Feldverteilung aus einer zweiten Nachschlagetabelle, wobei ein spezifisches physikalisches Merkmal, das mit der B1-Feldverteilung zusammenhängt, mit dem extrahierten physikalischen Merkmal übereinstimmt, und spezifische Projektionsdaten, die mit der B1-Feldverteilung zusammenhängen, mit den erfassten Projektionsdaten übereinstimmen; und
Bestimmen (1026) eines spezifischen Satzes von B1-Shimparametern, der mit der erhaltenen B1-Feldverteilung zusammenhängt, als der bestimmte Satz von B1-Shimparametern.

6. Shimverfahren (400) nach Anspruch 5, weiter umfassend:

Bestimmen (1062) von B1-Feldverteilungen jeweils für verschiedene entsprechende physikalische Merkmale;
Erzeugen (1064) entsprechender Projektionsdaten für jede der bestimmten B1-Feldverteilungen;
Bestimmen (1066) eines entsprechenden Satzes von B1-Shimparametern für jede der bestimmten B1-Feldverteilungen; und
Speichern (1068) der bestimmten B1-Feldverteilungen in der zweiten Nachschlagetabelle, wobei jede der bestimmten B1-Feldverteilungen in Verbindung mit dem entsprechenden physikalischen Merkmal, den entsprechenden Projektionsdaten und dem entsprechenden Satz von B1-Shimparametern gespeichert wird.

7. Verfahren (400) nach Anspruch 5 oder 6, wobei das extrahierte physikalische Merkmal mindestens eines von einer Dimensionsskala der Bildgebungsanatomie, einem Seitenverhältnis der Bildgebungsanatomie, einen Körperfettzusammensetzung des Patienten, einem Geschlecht des Patienten und einem Alter des Patienten einschließt.

8. Shimverfahren (400) nach einem der Ansprüche 2 bis 4, wobei der Schritt des Bestimmens des Satzes von B1-Shimparametern weiter umfasst:

Extrahieren eines spezifischen physikalischen Merkmals aus den Patientendaten;
Anwenden des extrahierten physikalischen Merkmals und der erfassten Projektionsdaten auf ein trainiertes neuronales Netzwerk; und
Bestimmen des Satzes von B1-Shimparametern aus den Ausgaben des neuronalen Netzes.

9. Shimverfahren (400) nach einem der Ansprüche 1 bis 4, wobei
der Schritt des Bestimmens (440) des Satzes von B1-Shimparametern weiter umfasst:

Empfangen (1210), entlang der bestimmten Ausrichtung, der Projektion, von B1-Projektionsdaten, die jeweils einer Vielzahl von Sätzen von B1-Shimparametern entsprechen;
Berechnen (1220), auf der Grundlage der empfangenen B1-Projektionsdaten, eines Kostenfunktionswerts, der jedem der Vielzahl von Sätzen von B1-Shimparametern entspricht;
Identifizieren (1230) eines spezifischen Satzes von B1-Shimparametern, der einem niedrigsten Kostenfunktionswert entspricht; und
Bestimmen (1240) des identifizierten spezifischen Satzes von B1-Shimparametern als der bestimmte Satz von B1-Shimparametern.

10. Shimverfahren (400) nach einem der Ansprüche 1 bis 4, wobei der Schritt des Bestimmens (440) des Satzes von B1-Shimparametern weiter umfasst:

Steuern (1610) eines Satzes von B1-Shimparametern zum Einschalten jedes einzelnen Sendekanals in einem HF-Sender des Magnetresonanztomographiesystems, wobei andere Sendekanäle ausgeschaltet sind;
Empfangen (1620) spezifischer B1-Projektionsdaten, die jeweils mit eingeschalteten einzelnen Sendekanälen erfasst wurden;
Analysieren (1630) der empfangenen spezifischen B1-Projektionsdaten, um einen Einfluss jedes einzelnen Sendekanals auf eine Symmetrie eines Profils der B1-Projektionsdaten zu bewerten;
Berechnen (1640) eines spezifischen Satzes von B1-Shimparametern, der die Symmetrie des Profils der B1-Projektionsdaten maximiert; und
Bestimmen (1640) des berechneten spezifischen Satzes von B1-Shimparametern als der bestimmte Satz von B1-Shimparametern.

11. Shimverfahren (400) nach Anspruch 10, wobei der Schritt des Bestimmens (420) der Ausrichtung der Projektion weiter das Bestimmen der Ausrichtung so umfasst, dass das Profil der entlang der bestimmten Ausrichtung erfassten B1-Projektionsdaten eine minimale Symmetrie aufweist.

12. Shimverfahren (400) nach einem vorstehenden Anspruch, wobei der Schritt des Erfassens (430) der B1-Projektionsdaten weiter mindestens einen der Folgenden umfasst:

Erfassen der Projektionsdaten unter Verwendung eines Bloch-Siegert-Shift-Verfahrens, eines Doppelwinkelverfahrens, eines Actual-Flip-Angle-Verfahrens, eines Dual-Refocusing-Echo-Acquisition-Mode-Verfahrens, eines phasensensitiven Verfahrens oder eines Sättigungswiederherstellungsverfahrens; und
Durchführen einer 2D-Raumauswahl zur Auswahl eines Abschnitts innerhalb eines Volumens des Patienten entlang der bestimmten Ausrichtung der Projektion.

13. Shimverfahren (400) nach einem vorstehenden Anspruch, wobei der Schritt des Erfassens (430) der B1-Projektionsdaten weiter das Durchführen von mehr als einem Auslesen pro Anregung umfasst, um mehr als eine Projektion pro Wiederholungszeit zu erfassen.

14. Shimeinrichtung (300) zum Durchführen eines patientenspezifischen B1-Feld-Shims in einem Magnetresonanztomographiesystem, wobei die Einrichtung umfasst:
Verarbeitungsschaltungen (310, 320, 330), die so konfiguriert sind, dass sie

Patienteninformationen über einen Patienten, der mit dem Magnetresonanztomographiesystem untersucht werden soll, erhalten;
eine Ausrichtung einer Projektion auf der Grundlage der erhaltenen Patienteninformationen bestimmen;
Bl-Projektionsdaten unter Verwendung des Magnetresonanztomographiesystems entlang der bestimmten Projektionsrichtung erfassen; und
einen Satz von B1-Shimparametern auf der Grundlage der erfassten B1-Projektionsdaten bestimmen; das Magnetresonanztomographiesystem auf der Grundlage des bestimmten Satzes von B1-Shimparametern steuern.

## Revendications

1. Procédé d'ajustement (400) pour réaliser un ajustement de champ B1 spécifique au patient dans un système d'imagerie par résonance magnétique, comprenant :

l'obtention (410) d'informations de patient d'un patient devant subir un examen d'imagerie par le système d'imagerie par résonance magnétique ;
la détermination (420) d'une orientation d'une projection sur la base des informations obtenues sur le patient ;
l'acquisition (430) de données de projection B1, à l'aide du système d'imagerie par résonance magnétique, selon l'orientation déterminée de la projection ;
la détermination (440) d'un ensemble de paramètres d'ajustement B1 sur la base des données de projection B1 acquises ;

la commande (450) du système d'imagerie par résonance magnétique sur la base de l'ensemble déterminé de paramètres d'ajustement B1.

2. Procédé d'ajustement (400) selon la revendication 1, dans lequel l'étape de détermination (420) de l'orientation de la projection comprend en outre :

l'extraction (622), à partir des informations de patient, d'une anatomie d'imagerie spécifique, et

l'obtention (624), à partir d'une première table de consultation, en utilisant l'anatomie d'imagerie extraite comme clé, de l'orientation de la projection et

l'étape d'acquisition (430) des données de projection B1 comprend en outre :

la génération (626) de signaux de gradient sur la base de l'orientation obtenue de la projection et l'application (628) des signaux de gradient générés aux pilotes de bobine de gradient du système d'imagerie par résonance magnétique.

3. Procédé d'ajustement (400) selon la revendication 2, comprenant en outre :

la détermination (662) de distributions de champ B1 correspondant respectivement à différentes anatomies d'imagerie particulières ;

la réalisation (664) d'une analyse en composantes principales sur chacune des distributions de champ B1 déterminées ;

l'identification (666), sur la base d'un résultat de l'analyse en composantes principales, d'une projection caractéristique pour chacune des distributions de champ B1 déterminées ; et

le stockage (668) des projections caractéristiques identifiées et des anatomies d'imagerie correspondantes sous forme de paires appariées dans la première table de consultation.

4. Procédé d'ajustement (400) selon la revendication 3, dans lequel l'étape de détermination (662) des distributions de champ B1 comprend en outre la détermination des distributions de champ B1 sur la base de données collectées dans une simulation physique, une expérience sur un fantôme, une expérience in vivo et/ou une procédure clinique pratiquée sur un patient.

5. Procédé d'ajustement (400) selon l'une quelconque des revendications 2 à 4, dans lequel l'étape de détermination (440) de l'ensemble de paramètres d'ajustement B1 comprend en outre :

l'extraction (1024), à partir des informations de patient, d'une caractéristique physique spécifique ;

l'obtention (1026), à partir d'une seconde table de consultation, d'une distribution de champ B1, dans lequel une caractéristique physique particulière liée à la distribution de champ B1 correspond à la caractéristique physique extraite et des données de projection particulières liées à la distribution de champ B1 correspondent aux données de projection acquises ; et

la détermination (1026) d'un ensemble particulier de paramètres d'ajustement B1 liés à la distribution de champ B1 obtenue pour être l'ensemble déterminé de paramètres d'ajustement B1.

6. Procédé d'ajustement (400) selon la revendication 5, comprenant en outre :

la détermination (1062) des distributions de champ B1 pour différentes caractéristiques physiques correspondantes, respectivement ;

la génération (1064) de données de projection correspondantes pour chacune des distributions de champ B1 déterminées ;

la détermination (1066) d'un ensemble correspondant de paramètres d'ajustement B1 pour chacune des distributions de champ B1 déterminées ; et

le stockage (1068) des distributions de champ B1 déterminées dans la seconde table de consultation, dans lequel chacune des distributions de champ B1 déterminées est stockée en association avec la caractéristique physique correspondante, les données de projection correspondantes et l'ensemble correspondant de paramètres d'ajustement B1.

7. Procédé (400) selon la revendication 5 ou la revendication 6, dans lequel la caractéristique physique extraite inclut au moins une parmi une échelle dimensionnelle de l'anatomie d'imagerie, un rapport d'aspect de l'anatomie d'imagerie, une composition corporelle en graisse du patient, un sexe du patient et un âge du patient.

8. Procédé d'ajustement (400) selon l'une quelconque des revendications 2 à 4, dans lequel l'étape de détermination de l'ensemble de paramètres d'ajustement B1 comprend en outre :

l'extraction, à partir des informations de patient, d'une caractéristique physique spécifique ;
l'application de la caractéristique physique extraite et des données de projection acquises à un réseau neuronal entraîné ; et
la détermination de l'ensemble de paramètres de réglage B1 à partir de sorties du réseau neuronal.

9. Procédé d'ajustement (400) selon l'une quelconque des revendications 1 à 4, dans lequel l'étape de détermination (440) de l'ensemble de paramètres d'ajustement B1 comprend en outre :

la réception (1210), le long de l'orientation déterminée de la projection, de données de projection B1 correspondant à chacun d'une pluralité d'ensembles de paramètres d'ajustement B1, respectivement;
le calcul (1220), sur la base des données de projection B1 reçues, d'un score de fonction de coût correspondant à chacun de la pluralité d'ensembles de paramètres d'ajustement B1, respectivement ;
l'identification (1230) d'un ensemble particulier de paramètres d'ajustement B1 qui correspond à un score de fonction de coût le plus bas ; et
la détermination (1240) de l'ensemble particulier identifié de paramètres d'ajustement B1 comme étant l'ensemble déterminé de paramètres d'ajustement B1.

10. Procédé d'ajustement (400) selon l'une quelconque des revendications 1 à 4, dans lequel l'étape de détermination (440) de l'ensemble de paramètres d'ajustement B1 comprend en outre :

la commande (1610) d'un ensemble de paramètres d'ajustement B1 pour activer chacun des canaux d'émission individuels dans un émetteur RF du système d'imagerie par résonance magnétique, les autres canaux d'émission étant désactivés ;
la réception (1620) de données de projection B1 particulières acquises avec chacun des canaux d'émission individuels activés, respectivement ;
l'analyse (1630) des données de projection B1 particulières reçues pour évaluer un effet de chacun des canaux d'émission individuels sur une symétrie d'un profil des données de projection B1 ;
le calcul (1640) d'un ensemble particulier de paramètres d'ajustement B1 qui maximise la symétrie du profil des données de projection B1; et
la détermination (1640) de l'ensemble particulier calculé de paramètres d'ajustement B1 comme étant l'ensemble déterminé de paramètres d'ajustement B1.

11. Procédé d'ajustement (400) selon la revendication 10, dans lequel l'étape de détermination (420) de l'orientation de la projection comprend en outre la détermination de l'orientation de telle sorte que le profil des données de projection B1 acquises le long de l'orientation déterminée présente une symétrie minimale.

12. Procédé d'ajustement (400) selon une quelconque revendication précédente, dans lequel l'étape d'acquisition (430) des données de projection B1 comprend en outre au moins l'une parmi :

l'acquisition des données de projection à l'aide d'un procédé de décalage de Bloch-Siegert, d'un procédé à double angle, d'un procédé d'angle de basculement réel, d'un procédé d'acquisition d'écho à double refocalisation, d'un procédé sensible à la phase ou d'un procédé de récupération de saturation ; et
la réalisation d'une sélection spatiale 2D pour sélectionner une portion dans un volume du patient selon l'orientation déterminée de la projection.

13. Procédé d'ajustement (400) selon une quelconque revendication précédente, dans lequel l'étape d'acquisition (430) des données de projection B1 comprend en outre la réalisation de plus d'une lecture par excitation pour acquérir plus d'une projection par temps de répétition.

14. Appareil d'ajustement (300) pour réaliser un ajustement de champ B1 spécifique au patient dans un système d'imagerie par résonance magnétique, l'appareil comprenant :
un circuit de traitement (310, 320, 330) configuré pour

obtenir des informations de patient d'un patient devant subir un examen d'imagerie par le système d'imagerie par résonance magnétique ;

déterminer une orientation d'une projection sur la base des informations obtenues sur le patient ;

acquérir des données de projection B1, à l'aide du système d'imagerie par résonance magnétique, selon l'orientation déterminée de la projection ; et

déterminer un ensemble de paramètres d'ajustement B1 sur la base des données de projection B1 acquises ;

commander le système d'imagerie par résonance magnétique sur la base de l'ensemble déterminé de paramètres d'ajustement B1.

# FIG.1

(a)

(b)

FIG.2

(a)

(b)

(c)

(d)

# FIG.3

to Gradient Coil Drivers          from RF Receiver

Patient
Information

| Projection Determination Circuitry 310 | Projection Data Acquisition Circuitry 320 | Shimming Parameter Resolving Circuitry 330 |

to RF Transmitter

300

EP 4 455 710 B1

# FIG.4

**400**

410 — Obtain patient information, including patient anatomy to be imaged

420 — Determine the orientation and number of projections on the basis of the patient anatomy

430 — Make the MRI acquire projection data along each of the determined projections

440 — Resolve a set of shimming parameters $\{A, \varphi\}$ based on the acquired projection data

450 — Apply the revolved shimming parameters $\{A, \varphi\}$ to the RF transmitter

# FIG.5

Patient Information →

EP 4 455 710 B1

to Gradient Coil Drivers

| Imaging Anatomy Extracting Circuitry 510 | → | Anatomy-Projection LUT 520 | → | Gradient Signal Generating Circuitry 530 |

310

**660**

662 — Perform EM simulation to obtain B1 distributions corresponding to different imaging anatomies

664 — Perform principal component analysis on each of the B1 distributions

666 — Identify characteristic projections for each of the B1 distributions

668 — Create and store matched anatomy-projection pairs in the anatomy-projection LUT

**FIG.6**

**620**

622 — Extract patient anatomy to be imaged from the patient information

624 — Search in the anatomy-projection LUT to derive projections that match with the imaging anatomy

626 — Generate gradient signals that are required to achieve each of the projections

628 — Apply the generated gradient signals to the gradient coil drivers

# FIG.7

EP 4 455 710 B1

# FIG.8

(a)

(b)

(c)

# FIG.9

from Projection Data Acquisition Circuitry

Patient Information

Physical Feature Extracting Circuitry 910

Projection-Distribution LUT 920

Shimming Parameter Determining Circuitry 930

to RF Transmitter

330

EP 4 455 710 B1

FIG.10

**1060**

1062 — Perform EM simulations to build B1 distributions with different physical features

1064 — Generate projection data for each of the B1 distributions

1066 — Solve a $\{A, \varphi\}$ solution for each of the B1 distributions

1068 — Store the B1 distributions in the projection-distribution LUT, each entry has its corresponding projection data, physical features, and $\{A, \varphi\}$ solution

**1020**

1022 — Receive projection data from the projection data acquisition circuitry

1024 — Extract patient physical features (including dimensional scales, the aspect ratio, etc.) from the obtained patient information

1026 — Search in the projection-distribution LUT to extract a B1 distribution that has matched projection data and matched physical features

1028 — Read and apply to the RF transmitter a set of shimming parameters $\{A, \varphi\}$ that corresponds to the extracted B1 distribution

# FIG.11

from Projection Data
Acquisition Circuitry

Separate Projection
Data Receiving
Circuitry 1110

Cost Function Score
Calculating Circuitry
1120

Shimming Parameter
Determining
Circuitry 1130

to RF Transmitter

330

# FIG.12

<u>**1200**</u>

1210 — Obtain separate projection data acquired with different sets of shimming parameters {A, φ} along one or more orientations

1220 — Calculate a cost function score for each of the different sets of shimming parameters {A, φ}

1230 — Identify a set of shimming parameters {A, φ} of which the cost function score is lowest

1240 — Apply the determined set of {A, φ}, as a finalized set of shimming parameters, to the RF transmitter

# FIG.13

(a)

(b)

(c)

(d)

FIG.14

# FIG.15

from Projection Data
Acquisition Circuitry

| Separate Projection Data Receiving Circuitry 1510 |
| Projection Profile Symmetry Analyzing Circuitry 1520 |
| Shimming Parameter Determining Circuitry 1530 |

to RF Transmitter

330

# FIG.16

_1600_

1610

Control the shimming parameters to make each of the transmit channels operate alone one after another

1620

Obtain separate projection data acquired with a different one of the transmit channels ON, the rest OFF

1630

Analyze the effect of each of the transmit channels on the symmetry of the projection profile

1640

Solve a set of {A, φ} that maximizes the symmetry of the projection profile

1650

Apply the solved set of {A, φ}, as finalized shimming parameters, to the RF transmitter

# FIG.17

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9086446 B